# EUROPEAN PATENT APPLICATION

(11) **EP 4 734 049 A2**
(43) Date of publication of application: **29.04.2026**
(21) Application number: 25210088.8
(22) Date of filing: 21.10.2025
(51) Int. Cl.: G06T 7/00

(54) **GASTROSCOPY AREA INDICATION SYSTEM AND METHOD**

(30) Priority: 28.10.2024 KR 20240148201; 11.12.2024 KR 20240183453
(71) Applicant: Waycen Inc., Seoul 06167 (KR)
(72) Inventor: KEUM, Jisoo, 16824 Yongin-si, Gyeonggi-do (KR); KIM, Kyung Nam, 16703 Suwon-si, Gyeonggi-do (KR)
(74) Representative: Viering, Jentschura & Partner mbB Patent- und Rechtsanwälte

(57) **Abstract**

Proposed is a gastroscopy area indication method that includes loading a gastroscopy image analysis model and setting an analysis condition of the analysis model by a model loading/condition setting part, initializing an analysis screen and displaying a picture of a normal stomach by a controller, preprocessing, by an image preprocessing part, a gastroscopy image received through an image receiving part so that subsequent image analysis is smoothly performed, analyzing the preprocessed gastroscopy image by an image analysis part by using the image analysis model based on AI, detecting and indicating, on the basis of a result of analysis by the image analysis part, at least one selected from a group of an examination area, a hernia area, and a lesion area in the gastroscopy image, and providing, by the controller, the result of analysis performed by the image analysis part.

## Description

### CROSS REFERENCE TO RELATED APPLICATION

The present application claims priority to Korean Patent Application No. 10-2024-0148201, filed 28 October 2024, and Korean Patent Application No. 10-2024-0183453, filed 11 December 2024, the entire contents of which are incorporated herein for all purposes by this reference.

### BACKGROUND OF THE INVENTION

### Field of the Invention

The present disclosure relates to a gastroscopy area indication system and method. In particular, the present disclosure relates to a gastroscopy area indication system and method that, in a situation such as a hernia in which an upper portion of the stomach enters into the thoracic cavity during a gastroscopy process, changes a picture of the stomach to a picture having the hernia, different from a normal stomach, and displays the picture having the hernia on an examination screen.

A Korean national project supported by Korean government associated with this invention is described below.
Project Serial Number P0026379
Government Department Ministry of Trade, Industry and Energy
Specialized Institution for Project Management Korea Institute for Advancement of Technology
Title of Research Business (24-25) Scale-up Technology Commercialization Program R&D Support (Phase 2)
Title of Project Advancement of Gastrointestinal Endoscopy Image Analysis System, and Development of Artificial Intelligence-Based Reporting System
Supervising Institute Waycen Inc. (Lead) / JOONSUNG IP&LAW FIRM. (Participant)
Research Period 01 January 2024 ~ 31 December 2025

### Description of the Related Art

In today's medical examinations, diagnosis of a neoplasm occurring in the stomach is generally primarily made by a physician finding the neoplasm through gastroscopy, and primarily determining whether gastric cancer is found considering the shape and the size of the inside of the stomach included in an endoscopic image. In addition, for a lesion suspected of being cancer among them, tissue is collected by performing gastroscopy, and a definitive diagnosis is often made through a pathological biopsy. However, in gastroscopy, a patient has to swallow an endoscope and the endoscope causes significant discomfort while passing through the esophagus to reach the stomach, and there is a possibility of complications such as esophageal perforation or gastric perforation. Therefore, it is necessary, for the benefit of the patient, to diagnose a gastric neoplasm while reducing the number of times gastroscopy is performed.

Therefore, rather than a physician performing gastroscopy to find a gastric neoplasm and analyzing a result of this and performing gastroscopy again for a biopsy, it is highly necessary to find a gastric neoplasm lesion in a gastroscopy image during a single gastroscopy examination, to evaluate a risk thereof in real time, to immediately determine whether to perform a biopsy on a lesion, and to perform a biopsy on a lesion having a cancer risk on the spot. Gradually, reducing the number of gastroscopy examinations in this way is a current trend. In evaluating a risk of a gastric neoplasm lesion in real time, if the risk is evaluated lower than an actual risk, a cancer lesion may be missed, resulting in a serious consequence that cancer treatment is not performed. If the risk is evaluated higher than the actual risk, an unnecessary biopsy may be performed, resulting in damage to a tissue of the patient.

However, a method of evaluating a risk of a gastric lesion by viewing a gastroscopy image in real time has not yet been established as a standard. Currently, such risk evaluation relies almost entirely on subjective judgment of a physician performing the gastroscopy. However, this method may lead to different diagnoses depending on experiences of physicians, and in regions where there is no physician having sufficient experience, there is a problem that an accurate diagnosis cannot be made.

### SUMMARY OF THE INVENTION

The present disclosure is directed to providing a gastroscopy area indication system and method being capable of enabling detailed indication of a stomach examination area by distinguishing between a normal stomach and a herniated stomach and indicating them on a screen, and of allowing a thorough gastroscopy by informing an examiner of hernia.

In addition, the present disclosure is directed to providing a gastroscopy area indication system and method capable of checking areas examined up to the present, of checking examination-omitted positions after completion of the examination, and of providing hernia information by indicating a main stomach examination area.

According to an embodiment of the present disclosure, there is provided a gastroscopy area indication system including:
a model loading/condition setting part configured to load a gastroscopy image analysis model, and set an analysis condition of the analysis model;
an image receiving part configured to receive a gastroscopy image frame;
an image preprocessing part configured to preprocess a gastroscopy image received through the image receiving part so that subsequent image analysis is smoothly performed;
an image analysis part configured to analyze the gastroscopy image preprocessed by the image preprocessing part by using the image analysis model based on artificial intelligence (AI), and detect and indicate, on the basis of a result of analysis, at least one selected from a group of an examination area, a hernia area, and a lesion area in the gastroscopy image; and
a controller configured to check states and control operations of the model loading/condition setting part, the image receiving part, the image preprocessing part, and the image analysis part, and initialize an analysis screen and displays a picture of a normal stomach when the model loading/condition setting part completes loading of the gastroscopy image analysis model and setting of the analysis condition of the analysis model, and provide the result of analysis performed by the image analysis part.

Herein, preprocessing of the gastroscopy image by the image preprocessing part may include analysis region cropping and input size adjustment.

In addition, the image analysis model of the image analysis part may be configured as a single image analysis model for detecting the examination area and the hernia area.

In addition, the image analysis model of the image analysis part may be configured to include an examination area detection model for detecting the examination area, and a hernia detection model for detecting the hernia area.

In addition, the image analysis model of the image analysis part may be configured to include an examination area detection model for detecting the examination area, a hernia detection model for detecting the hernia area, and a lesion detection model for detecting the lesion area.

Herein, the lesion detection model may have a lesion attribute identification function for determining whether a lesion is benign or malignant.

In addition, in detecting and indicating at least one selected from the group of the examination area, the hernia area, and the lesion area in the gastroscopy image by the image analysis part, the examination area may include the gastroesophageal junction, the gastric body, the gastric antrum, the gastric cardia, the gastric angle, the gastric fundus, and the duodenal bulb, and the hernia area may include an upper gastric hernia and a paraesophageal hernia.

In addition, in detecting and indicating at least one selected from the group of the examination area, the hernia area, and the lesion area in the gastroscopy image by the image analysis part, the controller may be configured to transmit, to the image analysis part, an indication condition change command to indicate the gastroesophageal junction in a case of an upper gastric hernia, or indicate the gastric cardia and the gastric fundus in a case of a paraesophageal hernia.

In addition, in detecting and indicating at least one selected from the group of the examination area, the hernia area, and the lesion area in the gastroscopy image by the image analysis part, the controller may be configured to transmit, to the image analysis part, an indication condition change command to differently indicate a hernia in the picture of the normal stomach, or not indicate the hernia in the picture of the normal stomach, or report a gastric hernia.

In addition, according to an embodiment of the present disclosure, there is provided a gastroscopy area indication method including:
a) loading, by a model loading/condition setting part, a gastroscopy image analysis model and setting an analysis condition of the analysis model;
b) initializing, by a controller, an analysis screen and displaying a picture of a normal stomach;
c) preprocessing, by an image preprocessing part, a gastroscopy image received through an image receiving part so that subsequent image analysis is smoothly performed;
d) analyzing, by an image analysis part, the preprocessed gastroscopy image using the image analysis model based on AI;
e) detecting and indicating, on the basis of a result of analysis by the image analysis part, at least one selected from a group of an examination area, a hernia area, and a lesion area in the gastroscopy image; and
f) providing, by the controller, the result of analysis performed by the image analysis part.

Herein, in the step c), preprocessing of the gastroscopy image by the image preprocessing part may include analysis region cropping and input size adjustment.

In addition, in the step d), the image analysis model may be configured as a single image analysis model for detecting the examination area and the hernia area.

In addition, in the step d), the image analysis model may be configured to include an examination area detection model for detecting the examination area, and a hernia detection model for detecting the hernia area.

In addition, in the step d), the image analysis model may be configured to include an examination area detection model for detecting the examination area, a hernia detection model for detecting the hernia area, and a lesion detection model for detecting the lesion area.

Herein, the lesion detection model may have a lesion attribute identification function for determining whether a lesion is benign or malignant.

In addition, in the step e), in detecting and indicating at least one selected from the group of the examination area, the hernia area, and the lesion area in the gastroscopy image by the image analysis part, the examination area may include the gastroesophageal junction, the gastric body, the gastric antrum, the gastric cardia, the gastric angle, the gastric fundus, and the duodenal bulb, and the hernia area may include an upper gastric hernia and a paraesophageal hernia.

In addition, in the step e), in detecting and indicating at least one selected from the group of the examination area, the hernia area, and the lesion area in the gastroscopy image by the image analysis part, the controller may be configured to transmit, to the image analysis part, an indication condition change command to indicate the gastroesophageal junction in a case of an upper gastric hernia, or indicate the gastric cardia and the gastric fundus in a case of a paraesophageal hernia.

In addition, in the step e), in detecting and indicating at least one selected from the group of the examination area, the hernia area, and the lesion area in the gastroscopy image by the image analysis part, the controller may be configured to transmit, to the image analysis part, an indication condition change command to differently indicate a hernia in the picture of the normal stomach, or not indicate the hernia in the picture of the normal stomach, or report a gastric hernia.

In addition, according to another embodiment of the present disclosure, there is provided a gastroscopy area indication system including:
a model loading/condition setting part configured to load a gastroscopy image analysis model and a speech keyword recognition model, and set an analysis condition of the analysis model;
an image receiving part configured to receive a gastroscopy image frame;
an image preprocessing part configured to preprocess a gastroscopy image received through the image receiving part so that subsequent image analysis is smoothly performed;
an image analysis part configured to analyze the gastroscopy image preprocessed by the image preprocessing part by using the image analysis model based on artificial intelligence (AI), and detect and indicate, on the basis of a result of analysis, at least one selected from a group of an examination area, a hernia area, and a lesion area in the gastroscopy image;
a speech recognition part configured to read audio from a buffer storing the audio while the image analysis part performs the image analysis, and analyze the audio using the speech keyword recognition model based on AI, and recognize a speech keyword on the basis of the result of analysis and transmit the speech keyword to the image analysis part; and
a controller configured to check states and control operations of the model loading/condition setting part, the image receiving part, the image preprocessing part, the image analysis part, and the speech recognition part, and initialize an analysis screen and display a picture of a normal stomach when the model loading/condition setting part completes loading of the gastroscopy image analysis model and setting of the analysis condition of the analysis model, and provide the result of analysis performed by the image analysis part, wherein the result of analysis is provided by linking an analysis target detected by the image analysis model with a speech command (keyword) related to the analysis target spoken by an examiner.

Herein, preprocessing of the gastroscopy image by the image preprocessing part may include analysis region cropping and input size adjustment.

In addition, the image analysis model of the image analysis part may be configured as a single image analysis model for detecting the examination area and the hernia area.

In addition, the image analysis model of the image analysis part may be configured to include an examination area detection model for detecting the examination area, and a hernia detection model for detecting the hernia area.

In addition, the image analysis model of the image analysis part may be configured to include an examination area detection model for detecting the examination area, a hernia detection model for detecting the hernia area, and a lesion detection model for detecting the lesion area.

Herein, the lesion detection model may have a lesion attribute identification function for determining whether a lesion is benign or malignant.

In addition, in detecting and indicating at least one selected from the group of the examination area, the hernia area, and the lesion area in the gastroscopy image by the image analysis part, the examination area may include the gastroesophageal junction, the gastric body, the gastric antrum, the gastric cardia, the gastric angle, the gastric fundus, and the duodenal bulb, and the hernia area may include an upper gastric hernia and a paraesophageal hernia.

In addition, in detecting and indicating at least one selected from the group of the examination area, the hernia area, and the lesion area in the gastroscopy image by the image analysis part, the controller may be configured to transmit, to the image analysis part, an indication condition change command to indicate the gastroesophageal junction in a case of an upper gastric hernia, or indicate the gastric cardia and the gastric fundus in a case of a paraesophageal hernia.

In addition, in detecting and indicating at least one selected from the group of the examination area, the hernia area, and the lesion area in the gastroscopy image by the image analysis part, the controller may be configured to transmit, to the image analysis part, an indication condition change command to differently indicate a hernia in the picture of the normal stomach, or not indicate the hernia in the picture of the normal stomach, or report a gastric hernia.

In addition, according to another embodiment of the present disclosure, there is provided a gastroscopy area indication method including:
p) loading, by a model loading/condition setting part, a gastroscopy image analysis model and a speech keyword recognition model and setting an analysis condition of the analysis model;
q) initializing, by a controller, an analysis screen and displaying a picture of a normal stomach;
r) preprocessing, by an image preprocessing part, a gastroscopy image received through an image receiving part so that subsequent image analysis is smoothly performed;
s) analyzing, by an image analysis part, the gastroscopy image preprocessed by the image preprocessing part using the image analysis model based on AI;
t) detecting and indicating, on the basis of a result of analysis by the image analysis part, at least one selected from a group of an examination area, a hernia area, and a lesion area in the gastroscopy image;
u) reading, by a speech recognition part, audio from a buffer storing the audio while the image analysis part performs the image analysis and analyzing the audio using the speech keyword recognition model based on AI, and recognizing a speech keyword on the basis of the result of analysis and transmitting the speech keyword to the image analysis part; and
v) providing, by the controller, the result of analysis by linking an analysis target detected by the image analysis model of the image analysis part with a speech command (keyword) related to the analysis target spoken by an examiner.

Herein, in the step r), preprocessing of the gastroscopy image by the image preprocessing part may include analysis region cropping and input size adjustment.

In addition, in the step s), the image analysis model may be configured as a single image analysis model for detecting the examination area and the hernia area.

In addition, in the step s), the image analysis model may be configured to include an examination area detection model for detecting the examination area, and a hernia detection model for detecting the hernia area.

In addition, in the step s), the image analysis model may be configured to include an examination area detection model for detecting the examination area, a hernia detection model for detecting the hernia area, and a lesion detection model for detecting the lesion area.

Herein, the lesion detection model may have a lesion attribute identification function for determining whether a lesion is benign or malignant.

In addition, in the step t), in detecting and indicating at least one selected from the group of the examination area, the hernia area, and the lesion area in the gastroscopy image by the image analysis part, the examination area may include the gastroesophageal junction, the gastric body, the gastric antrum, the gastric cardia, the gastric angle, the gastric fundus, and the duodenal bulb, and the hernia area may include an upper gastric hernia and a paraesophageal hernia.

In addition, in the step t), in detecting and indicating at least one selected from the group of the examination area, the hernia area, and the lesion area in the gastroscopy image by the image analysis part, the controller may be configured to transmit, to the image analysis part, an indication condition change command to indicate the gastroesophageal junction in a case of an upper gastric hernia, or indicate the gastric cardia and the gastric fundus in a case of a paraesophageal hernia.

In addition, in the step t), in detecting and indicating at least one selected from the group of the examination area, the hernia area, and the lesion area in the gastroscopy image by the image analysis part, the controller may be configured to transmit, to the image analysis part, an indication condition change command to differently indicate a hernia in the picture of the normal stomach, or not indicate the hernia in the picture of the normal stomach, or report a gastric hernia.

According to the present disclosure, a normal stomach and a herniated stomach are indicated in a screen by distinguishing therebetween, thereby specifically indicating a stomach examination area, and an examiner is informed of a hernia, thereby enabling a more thorough gastroscopy.

In addition, by indicating a main stomach examination area, it is possible to check areas examined up to the present, to check examination-omitted positions after completion of the examination, and to provide hernia information.

### BRIEF DESCRIPTION OF THE DRAWINGS

The above and other objectives, features, and other advantages of the present disclosure will be more clearly understood from the following detailed description when taken in conjunction with the accompanying drawings, in which:
FIG. 1 is a diagram schematically illustrating the configuration of a gastroscopy area indication system according to an embodiment of the present disclosure;
FIGS. 2A1, 2A2, 2B1, 2B2 and 2C are diagrams illustrating examples of configurations of an image analysis model;
FIG. 3 is a flowchart illustrating a process of performing a gastroscopy area indication method according to an embodiment of the present disclosure;
FIG. 4 is a flowchart illustrating a process of performing a first variation of the gastroscopy area indication method of FIG. 3;
FIG. 5 is a flowchart illustrating a process of performing a second variation of the gastroscopy area indication method of FIG. 3;
FIG. 6 is a flowchart illustrating a process of performing a third variation of the gastroscopy area indication method of FIG. 3;
FIG. 7 is a diagram schematically illustrating the configuration of a gastroscopy area indication system according to another embodiment of the present disclosure;
FIG. 8 is a flowchart illustrating a process of performing a gastroscopy area indication method according to another embodiment of the present disclosure;
FIG. 9 is a flowchart illustrating a process of performing a variation of a gastroscopy area indication method according to another embodiment of the present disclosure;
FIGS. 10A and 10B are diagrams illustrating a main area of the stomach;
FIGS. 11A, 11B, and 11C are diagrams illustrating a lateral side of the stomach;
FIG. 12 is a diagram illustrating the display of an examination area of the stomach (in the case of the normal stomach) ;
FIG. 13 is a diagram illustrating the display of an examination area of the stomach (in the case of the upper gastric hernia);
FIG. 14 is a diagram illustrating the display of an examination area of the stomach (in the case of the paraesophageal hernia);
FIG. 15 is a diagram illustrating another example of the display of an examination area of the stomach (in the case of the upper gastric hernia);
FIG. 16 is a diagram illustrating another example of the display of an examination area of the stomach (in the case of the paraesophageal hernia);
FIGS. 17A, 17B, and 17C are diagrams illustrating examples of providing a result of analysis (examples of an examination of the normal stomach);
FIGS. 18A, 18B, and 18C are diagrams illustrating examples of providing a result of analysis (examples of detection of an upper gastric hernia and a lesion);
FIGS. 19A, 19B, and 19C are diagrams illustrating examples of providing a result of analysis (examples of an examination of the paraesophageal hernia);
FIGS. 20A, 20B, 20C, and 20D are diagrams illustrating examples of providing a result of analysis (examples of result reports).

### DETAILED DESCRIPTION OF THE INVENTION

Hereinafter, an embodiment of the present disclosure will be described with reference to the accompanying drawings.

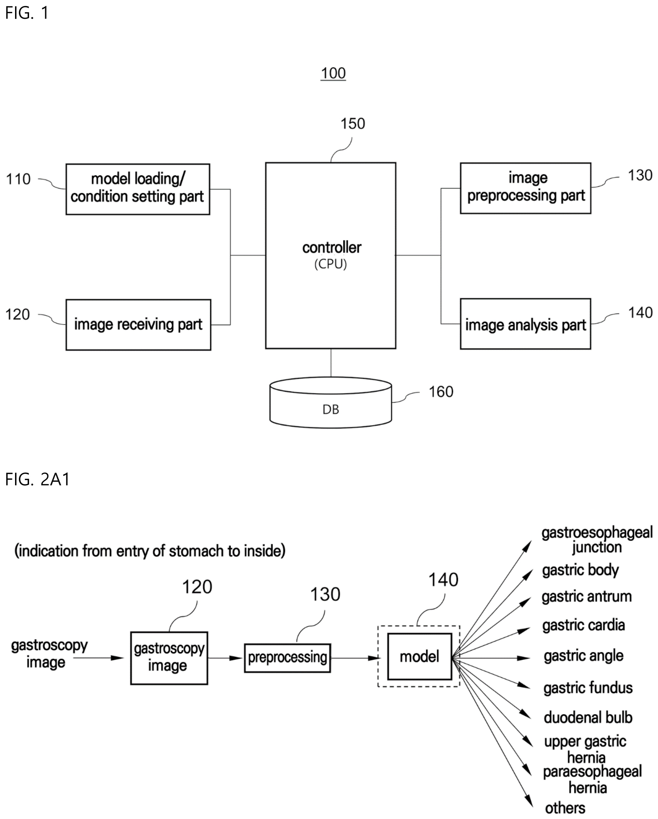

FIG. 1 is a diagram schematically illustrating the configuration of a gastroscopy area indication system according to an embodiment of the present disclosure.

Referring to FIG. 1, a gastroscopy area indication system 100 according to an embodiment of the present disclosure may include a model loading/condition setting part 110, an image receiving part 120, an image preprocessing part 130, an image analysis part 140, and a controller 150.

The model loading/condition setting part 110 loads a gastroscopy image analysis model and sets an analysis condition of the analysis model. Herein, the analysis condition of the analysis model may be set, for example, to a predicted probability value of 0.85 or higher.

The image receiving part 120 receives a gastroscopy image frame.

The image preprocessing part 130 preprocesses a gastroscopy image received through the image receiving part 120 so that subsequent image analysis is smoothly performed. Herein, the preprocessing of the gastroscopy image by the image preprocessing part 130 as described above may include analysis region cropping and input size adjustment.

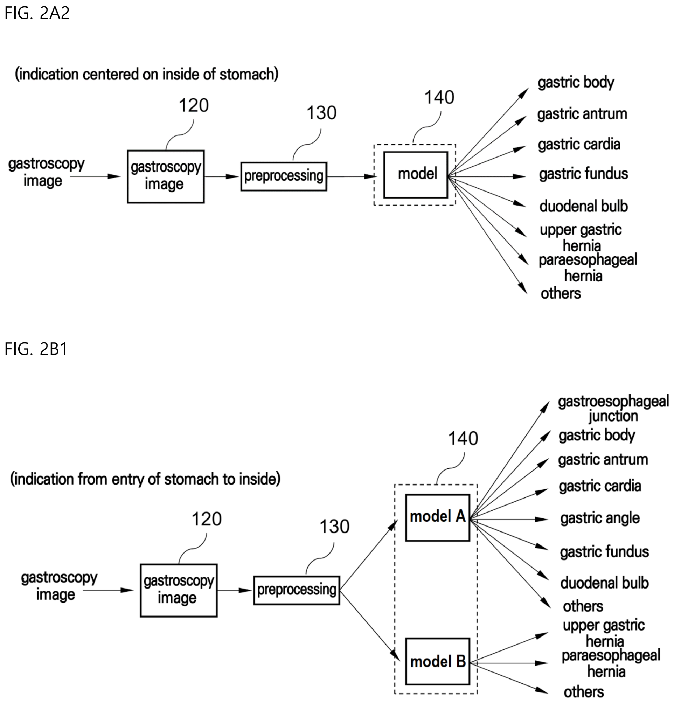

The image analysis part 140 analyzes the gastroscopy image preprocessed by the image preprocessing part 130 by using the image analysis model based on artificial intelligence (AI), and detects and indicates, on the basis of a result of analysis, at least one selected from the group of an examination area, a hernia area, and a lesion area in the gastroscopy image. Herein, the image analysis model of the image analysis part 140 may be configured as a single image analysis model for detecting an examination area and a hernia area as shown in FIGS. 2A1 and 2A2. FIG. 2A1 shows an example of indication from the entry of the stomach to the inside of the stomach, and FIG. 2A2 shows an example of indication centered on the inside of the stomach.

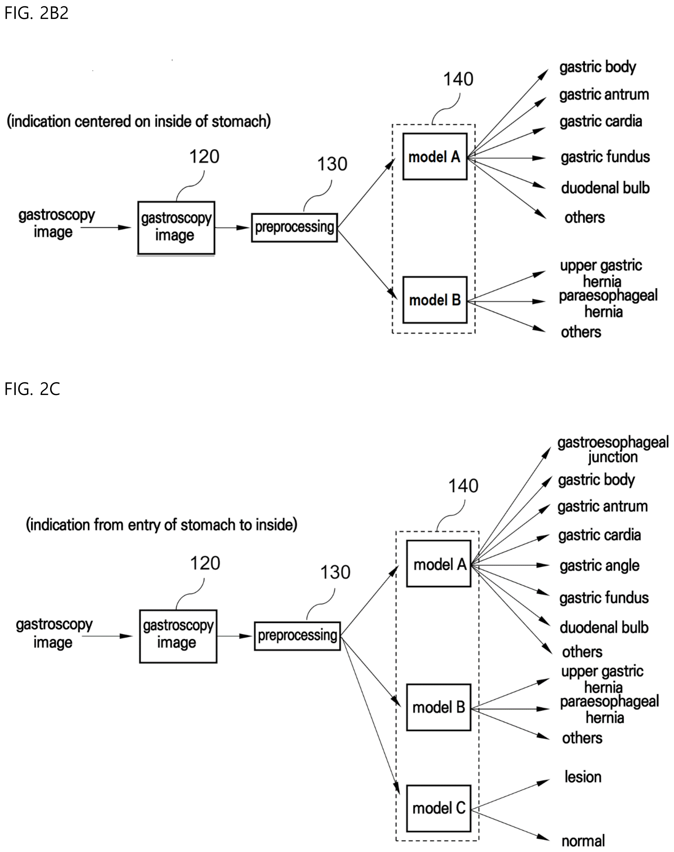

In addition, as shown in FIGS. 2B1 and 2B2, the image analysis model of the image analysis part 140 may include an examination area detection model (model A) for detecting an examination area, and a hernia detection model (model B) for detecting a hernia area. In FIGS. 2B1 and 2B2, FIG. 2B1 shows an example of indication from the entry of the stomach to the inside of the stomach, and FIG. 2B2 shows an example of indication centered on the inside of the stomach.

In addition, as shown in FIG. 2C, the image analysis model of the image analysis part 140 may include an examination area detection model (model A) for detecting an examination area, a hernia detection model (model B) for detecting a hernia area, and a lesion detection model (model C) for detecting a lesion area. Herein, the lesion detection model (model C) may include a lesion attribute identification function for determining whether the lesion is benign or malignant.

In addition, in detecting and indicating at least one selected from the group of an examination area, a hernia area, and a lesion area in the gastroscopy image by the image analysis part 140, the examination area may include the gastroesophageal junction, the gastric body, the gastric antrum, the gastric cardia, the gastric angle, the gastric fundus, and the duodenal bulb, and the hernia area may include an upper gastric hernia and a paraesophageal hernia.

The controller 150 checks the states and controls the operations of the model loading/condition setting part 110, the image receiving part 120, the image preprocessing part 130, and the image analysis part 140. When the model loading/condition setting part 110 completes the loading of the gastroscopy image analysis model and the setting of the analysis condition of the analysis model, the controller initializes an analysis screen, displays a picture of a normal stomach, and provides a result of analysis performed by the image analysis part 140. Herein, in detecting and indicating at least one selected from the group of an examination area, a hernia area, and a lesion area in the gastroscopy image by the image analysis part 140, the controller 150 may transmit, to the image analysis part 140, an indication condition change command to indicate the gastroesophageal junction in the case of the upper gastric hernia or indicate the gastric cardia and the gastric fundus in the case of the paraesophageal hernia. In addition, in detecting and indicating at least one selected from the group of an examination area, a hernia area, and a lesion area in the gastroscopy image by the image analysis part 140, the controller 150 may transmit, to the image analysis part 140, an indication condition change command to differently indicate the hernia in the picture of the normal stomach, to not indicate the hernia in the picture of the normal stomach, or to report the gastric hernia (for example, the alarm or the indication of the region).

In FIG. 1, reference numeral 160 denotes a database (DB). The database (DB) 160 stores and manages various software programs for system operation, as well as data or information required when the model loading/condition setting part 110, the image receiving part 120, the image preprocessing part 130, and the image analysis part 140 perform functions or process tasks related to model loading and condition setting, image preprocessing, and image analysis, and data on a result of gastroscopy image analysis performed by the image analysis model.

Herein, the model loading/condition setting part 110, the image receiving part 120, the image preprocessing part 130, the image analysis part 140, the controller 150, and the database (DB) 160 may be integrated as a whole and configured as a single computer system.

Hereinafter, a gastroscopy area indication method based on a gastroscopy area indication system having the configuration as described above according to an embodiment of the present disclosure will be described.

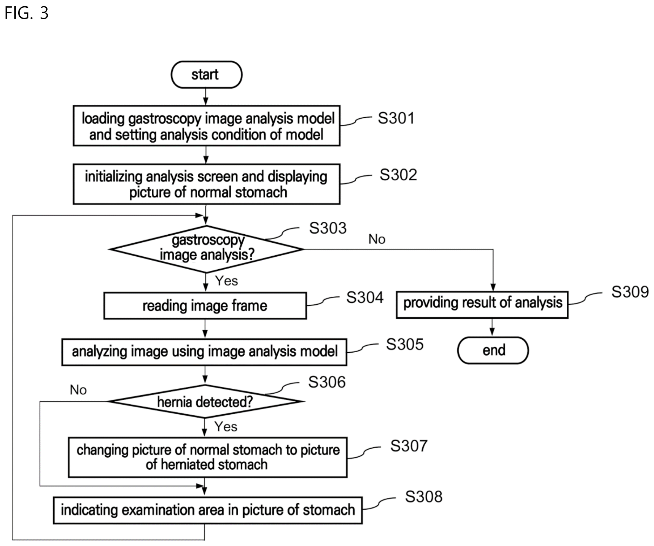

FIG. 3 is a flowchart illustrating a process of performing a gastroscopy area indication method according to an embodiment of the present disclosure.

Referring to FIG. 3, in a gastroscopy area indication method according to an embodiment of the present disclosure, first, the model loading/condition setting part 110 loads a gastroscopy image analysis model, and sets an analysis condition (for example, a predicted probability value of 0.85 or higher) of the analysis model in step S301.

Then, the controller 150 initializes an analysis screen and displays a picture of a normal stomach in step S302.

As described above, after the loading of the model and the setting of the analysis condition are completed, the analysis screen is initialized and the picture of the normal stomach is displayed, and then the controller 150 determines whether to perform gastroscopy image analysis in step S303. When gastroscopy image analysis is required as determined, the image preprocessing part 130 reads a gastroscopy image (image frame) received through the image receiving part 120, and preprocesses the gastroscopy image so that subsequent image analysis is smoothly performed in step S304. Herein, the preprocessing of the gastroscopy image by the image preprocessing part 130 may include analysis region cropping and input size adjustment.

When the preprocessing of the gastroscopy image is completed in this manner, the image analysis part 140 analyzes the preprocessed gastroscopy image using the image analysis model based on AI in step S305. Herein, the image analysis model may be configured as a single image analysis model for detecting an examination area and a hernia area, as shown in FIGS. 2A1 and 2A2 as described above. In addition, as shown in FIGS. 2B1 and 2B2, the image analysis model may include an examination area detection model (model A) for detecting an examination area, and a hernia detection model (model B) for detecting a hernia area. In addition, as shown in FIG. 2C, the image analysis model may include an examination area detection model (model A) for detecting an examination area, a hernia detection model (model B) for detecting a hernia area, and a lesion detection model (model C) for detecting a lesion area. Herein, the lesion detection model (model C) may include a lesion attribute identification function for determining whether the lesion is benign or malignant.

In addition, the image analysis part 140 detects and indicates, on the basis of a result of analysis, at least one selected from the group of an examination area, a hernia area, and a lesion area in the gastroscopy image in steps S306 to S308. Herein, in detecting and indicating at least one selected from the group of an examination area, a hernia area, and a lesion area in the gastroscopy image by the image analysis part 140, the examination area may include the gastroesophageal junction, the gastric body, the gastric antrum, the gastric cardia, the gastric angle, the gastric fundus, and the duodenal bulb, and the hernia area may include an upper gastric hernia and a paraesophageal hernia.

Herein, steps S306 to S308 will be described in more detail.

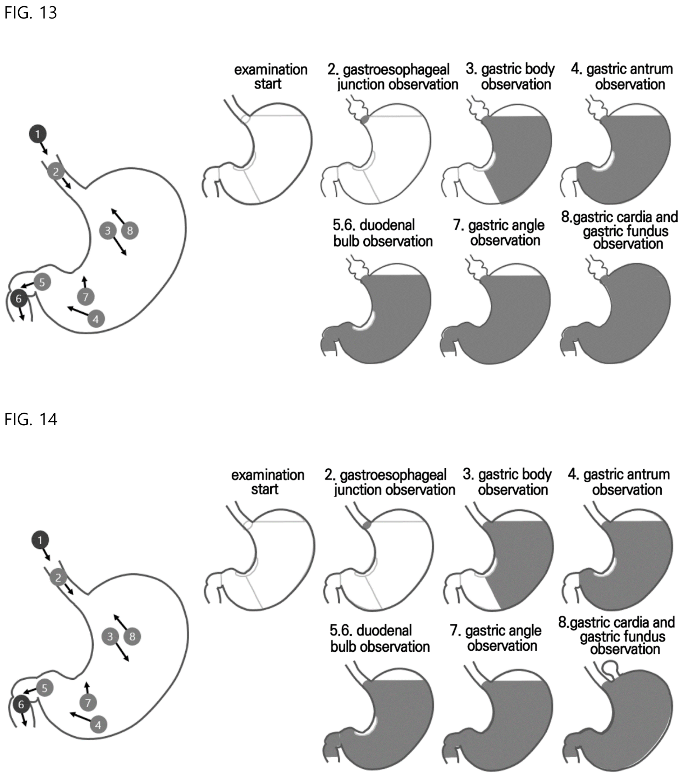

When the gastroscopy image analysis is completed by the image analysis part 140 in step S305, the controller 150 determines whether a hernia is detected in step S306. When a hernia is detected, the controller changes the picture of the normal stomach to a picture of a herniated stomach and displays the picture of the herniated stomach (see FIGS. 13 to 16) in step S307, and indicates an examination area in the picture of the stomach in step S308.

Afterward, when no further gastroscopy image analysis is required as determined in step S303, the controller 150 provides a result of the most recently performed analysis by the image analysis part 140 in step S309.

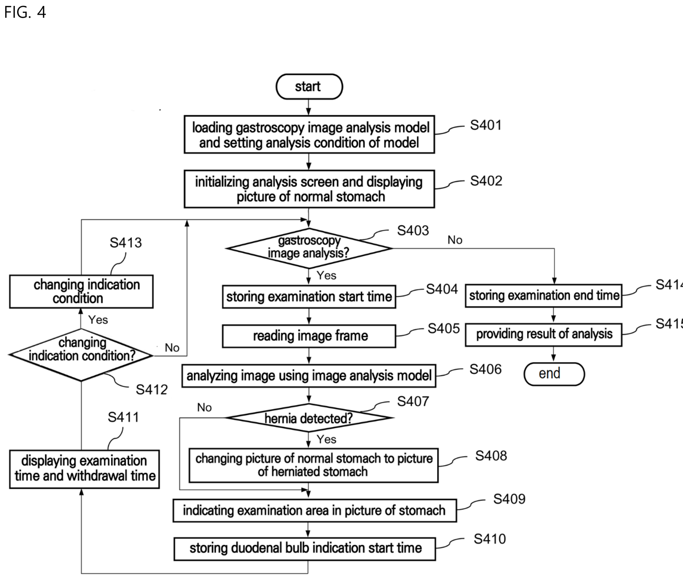

In the meantime, FIG. 4 is a flowchart illustrating a process of performing a first variation of the gastroscopy area indication method of FIG. 3.

Referring to FIG. 4, the process is identical to that of FIG. 3 described above except that the process of FIG. 4 further includes storing the examination start time in step S404, storing the duodenal bulb indication start time in step S410, displaying the examination time and the withdrawal time in step S411, determining whether to change an indication condition in step S412, changing the indication condition in step S413, and storing the examination end time in step S414. Therefore, the description of the portions that are identical to those in FIG. 3 will be replaced by the description of the portions of FIG. 3, and only the portions different from FIG. 3 will be described.

When gastroscopy image analysis is required as determined in step S403 of FIG. 4, the controller 150 stores the examination start time in the database 160 in step S404.

Afterward, as described above with reference to FIG. 3, the image preprocessing part 130 reads a gastroscopy image (image frame) received through the image receiving part 120, and preprocesses the gastroscopy image so that subsequent image analysis is smoothly performed in step S405.

In addition, the controller 150 indicates the examination area in the picture of the stomach in step S409, and stores the duodenal bulb indication start time in step S410. Afterward, the controller 150 displays the examination time and the withdrawal time in step S411. Herein, the examination time refers to the time from the examination start time to the current (examination end) time, and the withdrawal time refers to the time from the duodenal bulb indication start time to the current (examination end) time.

In addition, the controller 150 determines whether to change the indication condition in step S412, and changes the indication condition when the changing of the indication condition is required in step S413. Herein, the controller 150 may transmit, to the image analysis part 140, an indication condition change command to indicate the gastroesophageal junction in the case of the upper gastric hernia or indicate the gastric cardia and the gastric fundus in the case of the paraesophageal hernia.

In addition, the controller 150 may transmit, to the image analysis part 140, an indication condition change command to differently indicate the hernia in the picture of the normal stomach, to not indicate the hernia in the picture of the normal stomach, or to report the gastric hernia (for example, the alarm or the indication of the region).

In the meantime, when no further gastroscopy image analysis is required as determined in step S403, the controller 150 stores the examination end time in step S414, and provides a result of analysis up to the current (examination end) time in step S415.

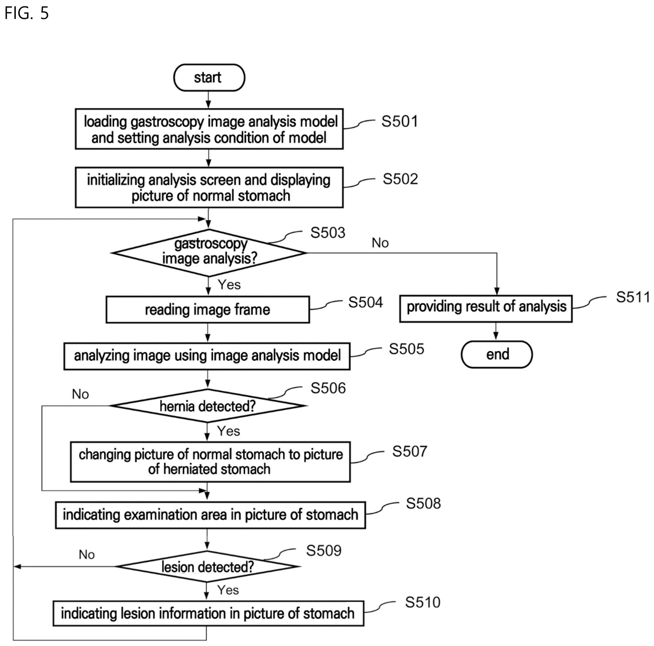

FIG. 5 is a flowchart illustrating a process of performing a second variation of the gastroscopy area indication method of FIG. 3.

Referring to FIG. 5, the process is identical to that of FIG. 3 described above except that the process of FIG. 5 further includes determining whether a lesion is detected in step S509, and indicating lesion information in the picture of the stomach in step S510. Therefore, the description of the portions that are identical to those in FIG. 3 will be replaced by the description of the portions of FIG. 3, and only the portions different from FIG. 3 will be described.

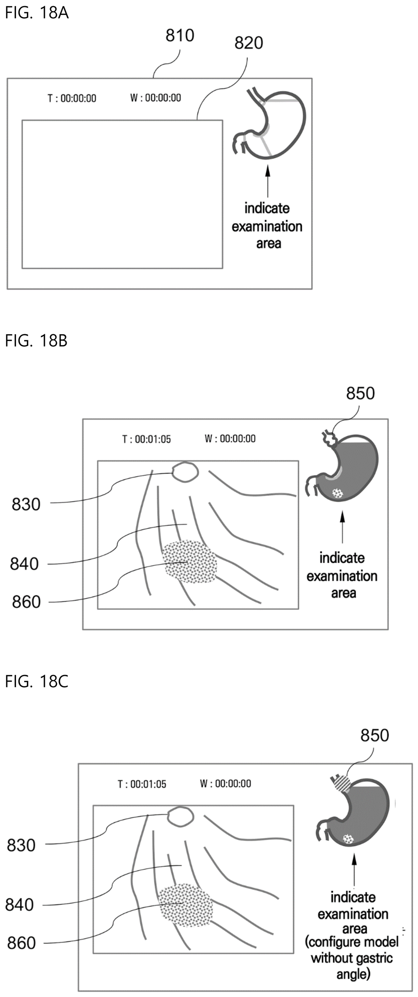

In FIG. 5, the controller 150 indicates the examination area in the picture of the stomach in step S508, and determines whether a lesion is detected in step S509, and indicates lesion information (see FIGS. 18A, 18B, and 18C) in the picture of the stomach when the lesion is detected in step S510.

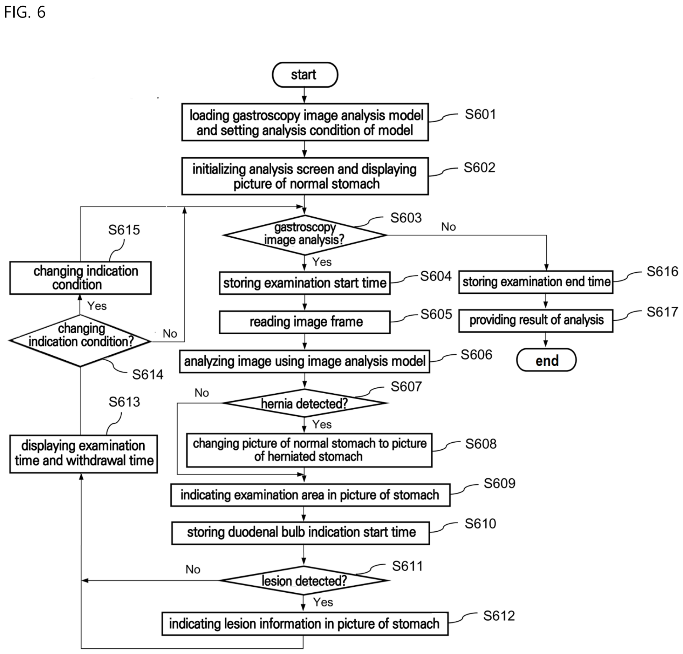

FIG. 6 is a flowchart illustrating a process of performing a third variation of the gastroscopy area indication method of FIG. 3.

Referring to FIG. 6, the process is identical to that of FIG. 4 described above except that the process of FIG. 6 further includes determining whether a lesion is detected in step S611, and indicating lesion information in the picture of the stomach in step S612.

That is, when gastroscopy image analysis is required as determined in step S603 of FIG. 6, the controller 150 stores the examination start time in the database 160 in step S604.

Afterward, as described above with reference to FIG. 3, the image preprocessing part 130 reads a gastroscopy image (image frame) received through the image receiving part 120, and preprocesses the gastroscopy image so that subsequent image analysis is smoothly performed in step S605.

In addition, the controller 150 indicates the examination area in the picture of the stomach in step S609, and stores the duodenal bulb indication start time in step S610. Afterward, the controller 150 determines whether a lesion is detected in step S611, and indicates lesion information (see FIGS. 18A, 18B, and 18C) in the picture of the stomach when the lesion is detected in step S612.

Next, the controller 150 displays the examination time and the withdrawal time in step S613. Herein, the examination time refers to the time from the examination start time to the current (examination end) time, and the withdrawal time refers to the time from the duodenal bulb indication start time to the current (examination end) time.

In addition, the controller 150 determines whether to change the indication condition in step S614, and changes the indication condition when the changing of the indication condition is required in step S615. Herein, the controller 150 may transmit, to the image analysis part 140, an indication condition change command to indicate the gastroesophageal junction in the case of the upper gastric hernia or indicate the gastric cardia and the gastric fundus in the case of the paraesophageal hernia.

In addition, the controller 150 may transmit, to the image analysis part 140, an indication condition change command to differently indicate the hernia in the picture of the normal stomach, to not indicate the hernia in the picture of the normal stomach, or to report the gastric hernia (for example, the alarm or the indication of the region).

In the meantime, when no further gastroscopy image analysis is required as determined in step S603, the controller 150 stores the examination end time in step S616, and provides a result of analysis up to the current (examination end) time in step S617.

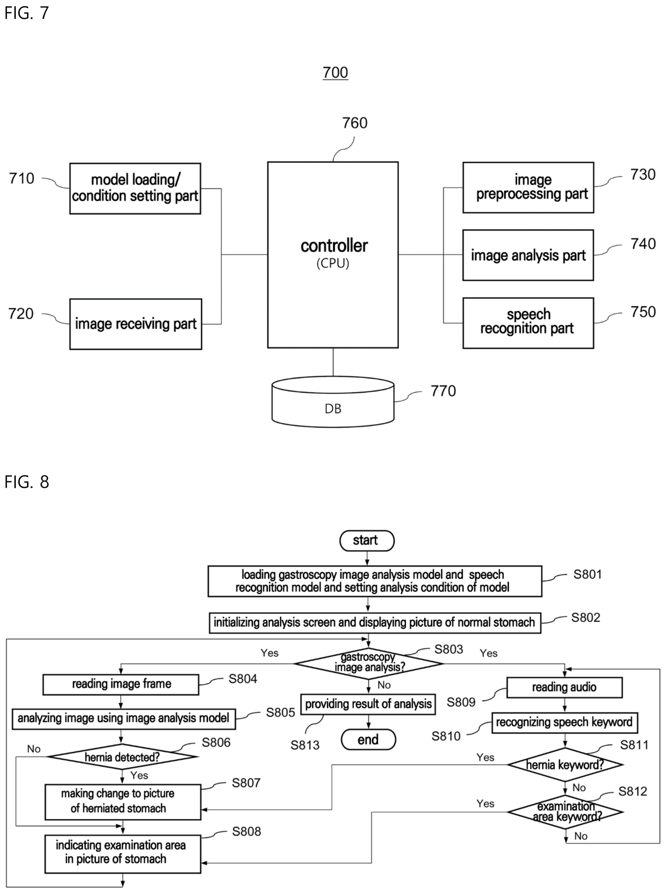

FIG. 7 is a diagram schematically illustrating the configuration of a gastroscopy area indication system according to another embodiment of the present disclosure.

Referring to FIG. 7, a gastroscopy area indication system 700 according to another embodiment of the present disclosure includes, fundamentally, the same elements as the gastroscopy area indication system 100 according to an embodiment described above with reference to FIG. 1. However, there is a difference in that the gastroscopy area indication system 700 according to the embodiment further includes a speech recognition part 750.

As shown in FIG. 7, a gastroscopy area indication system 700 according to another embodiment of the present disclosure may include a model loading/condition setting part 710, an image receiving part 720, an image preprocessing part 730, an image analysis part 740, a speech recognition part 750, and a controller 760.

The model loading/condition setting part 710 loads a gastroscopy image analysis model and sets an analysis condition of the analysis model. Herein, the analysis condition of the analysis model may be set, for example, to a predicted probability value of 0.85 or higher.

The image receiving part 720 receives a gastroscopy image frame.

The image preprocessing part 730 preprocesses a gastroscopy image received through the image receiving part 720 so that subsequent image analysis is smoothly performed. Herein, the preprocessing of the gastroscopy image by the image preprocessing part 730 as described above may include analysis region cropping and input size adjustment.

The image analysis part 740 analyzes the gastroscopy image preprocessed by the image preprocessing part 730 by using the image analysis model based on artificial intelligence (AI), and detects and indicates, on the basis of a result of analysis, at least one selected from the group of an examination area, a hernia area, and a lesion area in the gastroscopy image. Herein, the image analysis model of the image analysis part 740 may be configured as a single image analysis model for detecting an examination area and a hernia area as shown in FIGS. 2A1 and 2A2. FIG. 2A1 shows an example of indication from the entry of the stomach to the inside of the stomach, and FIG. 2A2 shows an example of indication centered on the inside of the stomach.

In addition, as shown in FIGS. 2B and 2B2, the image analysis model of the image analysis part 740 may include an examination area detection model (model A) for detecting an examination area, and a hernia detection model (model B) for detecting a hernia area. FIG. 2B1 shows an example of indication from the entry of the stomach to the inside of the stomach, and FIG. 2B2 shows an example of indication centered on the inside of the stomach.

In addition, as shown in FIG. 2C, the image analysis model of the image analysis part 740 may include an examination area detection model (model A) for detecting an examination area, a hernia detection model (model B) for detecting a hernia area, and a lesion detection model (model C) for detecting a lesion area. Herein, the lesion detection model (model C) may include a lesion attribute identification function for determining whether the lesion is benign or malignant.

In addition, in detecting and indicating at least one selected from the group of an examination area, a hernia area, and a lesion area in the gastroscopy image by the image analysis part 740, the examination area may include the gastroesophageal junction, the gastric body, the gastric antrum, the gastric cardia, the gastric angle, the gastric fundus, and the duodenal bulb, and the hernia area may include an upper gastric hernia and a paraesophageal hernia.

The speech recognition part 750 reads audio from a buffer (located in an internal memory of the speech recognition part 750), which stores audio, while the image analysis part 740 performs image analysis, and analyzes the audio using an AI-based speech keyword recognition model, and recognizes a speech keyword on the basis of a result of analysis and transmits the speech keyword to the image analysis part 740.

The controller 760 checks the states and controls the operations of the model loading/condition setting part 710, the image receiving part 720, the image preprocessing part 730, the image analysis part 740, and the speech recognition part 750. When the model loading/condition setting part 710 completes the loading of the gastroscopy image analysis model and the setting of the analysis condition of the analysis model, the controller initializes an analysis screen, displays a picture of a normal stomach, and provides a result of analysis performed by the image analysis part 740. The controller provides the result of analysis by linking an analysis target detected by the image analysis model with a speech command (keyword) related to the analysis target spoken by an examiner.

Herein, in detecting and indicating at least one selected from the group of an examination area, a hernia area, and a lesion area in the gastroscopy image by the image analysis part 740, the controller 760 may transmit, to the image analysis part 740, an indication condition change command to indicate the gastroesophageal junction in the case of the upper gastric hernia or indicate the gastric cardia and the gastric fundus in the case of the paraesophageal hernia. In addition, in detecting and indicating at least one selected from the group of an examination area, a hernia area, and a lesion area in the gastroscopy image by the image analysis part 740, the controller 760 may transmit, to the image analysis part 740, an indication condition change command to differently indicate the hernia in the picture of the normal stomach, to not indicate the hernia in the picture of the normal stomach, or to report the gastric hernia (for example, the alarm or the indication of the region).

In FIG. 7, reference numeral 770 denotes a database (DB). The database (DB) 770 stores and manages various software programs for system operation, as well as data or information required when the model loading/condition setting part 710, the image receiving part 720, the image preprocessing part 730, the image analysis part 740, and the speech recognition part 750 perform functions or process tasks related to model loading and condition setting, image preprocessing, image analysis, and speech recognition, and data on a result of gastroscopy image analysis performed by the image analysis model.

Herein, the model loading/condition setting part 710, the image receiving part 720, the image preprocessing part 730, the image analysis part 740, the speech recognition part 750, the controller 760, and the database (DB) 770 may be integrated as a whole and configured as a single computer system.

Hereinafter, a gastroscopy area indication method based on a gastroscopy area indication system having the configuration as described above according to another embodiment of the present disclosure will be described.

FIG. 8 is a flowchart illustrating a process of performing a gastroscopy area indication method according to another embodiment of the present disclosure.

Referring to FIG. 8, in a gastroscopy area indication method according to another embodiment of the present disclosure, first, the model loading/condition setting part 710 loads a gastroscopy image analysis model and a speech recognition model, and sets an analysis condition (for example, a predicted probability value of 0.85 or higher) of the analysis model in step S801.

Then, the controller 760 initializes an analysis screen and displays a picture of a normal stomach in step S802.

As described above, after the loading of the gastroscopy image analysis model, the loading of the speech recognition model, and the setting of the analysis condition of the model are completed, the analysis screen is initialized and the picture of the normal stomach is displayed, and then the controller 760 determines whether to perform gastroscopy image analysis in step S803. When gastroscopy image analysis is required as determined, the image preprocessing part 730 reads a gastroscopy image (image frame) received through the image receiving part 720, and preprocesses the gastroscopy image so that subsequent image analysis is smoothly performed in step S804. Herein, the preprocessing of the gastroscopy image by the image preprocessing part 730 may include analysis region cropping and input size adjustment.

When the preprocessing of the gastroscopy image is completed in this manner, the image analysis part 740 analyzes the preprocessed gastroscopy image using the image analysis model based on AI in step S805. Herein, the image analysis model may be configured as a single image analysis model for detecting an examination area and a hernia area, as shown in FIGS. 2A1 and 2A2 as described above. In addition, as shown in FIGS. 2B and 2B2, the image analysis model may include an examination area detection model (model A) for detecting an examination area, and a hernia detection model (model B) for detecting a hernia area. In addition, as shown in FIG. 2C, the image analysis model may include an examination area detection model (model A) for detecting an examination area, a hernia detection model (model B) for detecting a hernia area, and a lesion detection model (model C) for detecting a lesion area. Herein, the lesion detection model (model C) may include a lesion attribute identification function for determining whether the lesion is benign or malignant.

In addition, the image analysis part 740 detects and indicates, on the basis of a result of analysis, at least one selected from the group of an examination area, a hernia area, and a lesion area in the gastroscopy image in steps S806 to S808. Herein, in detecting and indicating at least one selected from the group of an examination area, a hernia area, and a lesion area in the gastroscopy image by the image analysis part 740, the examination area may include the gastroesophageal junction, the gastric body, the gastric antrum, the gastric cardia, the gastric angle, the gastric fundus, and the duodenal bulb, and the hernia area may include an upper gastric hernia and a paraesophageal hernia.

Herein, steps S806 to S808 will be described in more detail.

When the gastroscopy image analysis is completed by the image analysis part 740 in step S805, the controller 760 determines whether a hernia is detected in step S806. When a hernia is detected, the controller changes the picture of the normal stomach to a picture of a herniated stomach and displays the picture of the herniated stomach (see FIGS. 13 to 16) in step S807, and indicates an examination area in the picture of the stomach in step S808.

In the meantime, when gastroscopy image analysis is required as determined in step S803, the speech recognition part 750 reads audio from the buffer storing audio while the image analysis part 740 performs image analysis, and analyzes the audio using the AI-based speech keyword recognition model in step S809, and recognizes a speech keyword on the basis of a result of analysis in step S810, and transmits the speech keyword to the image analysis part 740.

That is, the speech recognition part 750 recognizes the speech keyword and determines whether the speech keyword is a hernia keyword in step S811. When the speech keyword is the hernia keyword, proceeding to step S807 takes place for change into a picture of a herniated stomach.

In addition, when the speech keyword is not the hernia keyword as determined above, the speech recognition part 750 determines whether the speech keyword is an examination area keyword in step S812. When the speech keyword is the examination area keyword, proceeding to step S808 takes place to indicate the examination area in the picture of the stomach.

Afterward, when no further gastroscopy image analysis is required as determined in step S803, the controller 760 links the analysis target detected by the image analysis model of the image analysis part with the speech command (keyword) related to the analysis target spoken by an examiner, and provides a result of analysis performed up to the current (examination end) time by the image analysis part 740 in step S813.

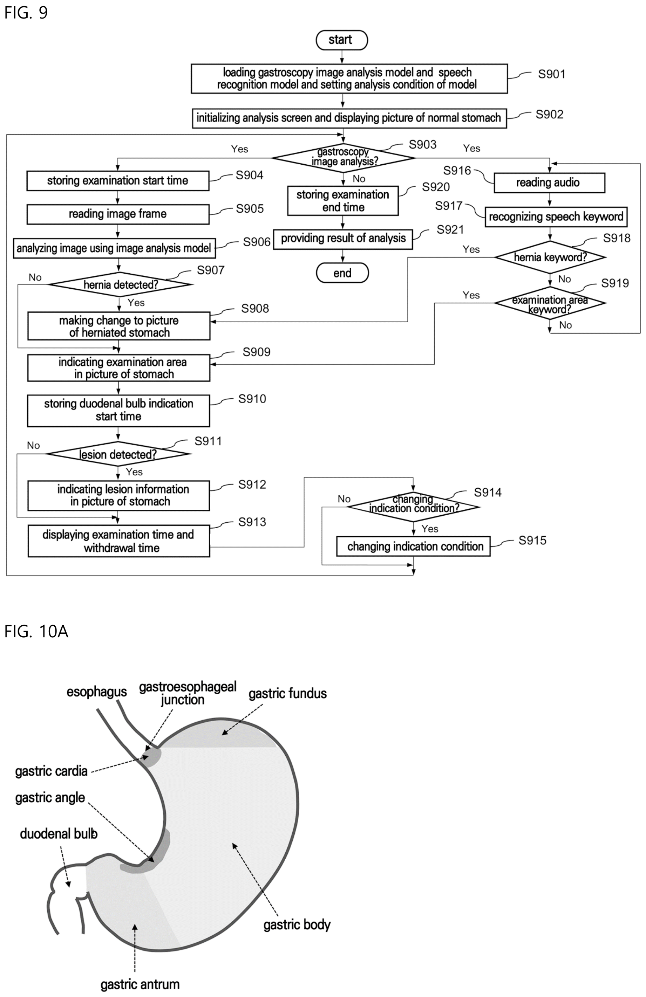

FIG. 9 is a flowchart illustrating a process of performing a variation of a gastroscopy area indication method according to another embodiment of the present disclosure.

Referring to FIG. 9, the process is identical to that of FIG. 8 described above except that the process of FIG. 9 further includes storing the examination start time in step S904, storing the duodenal bulb indication start time in step S910, determining whether a lesion is detected in step S911, indicating lesion information in the picture of the stomach in step S912, displaying the examination time and the withdrawal time in step S913, determining whether to change the indication condition in step S914, changing the indication condition in step S915, and storing the examination end time in step S920. Therefore, the description of the portions that are identical to those in FIG. 8 will be replaced by the description of the portions of FIG. 8, and only the portions different from FIG. 8 will be described.

When gastroscopy image analysis is required as determined in step S903 of FIG. 9, the controller 760 stores the examination start time in the database 770 in step S904.

Afterward, as described above with reference to FIG. 8, the image preprocessing part 730 reads a gastroscopy image (image frame) received through the image receiving part 720, and preprocesses the gastroscopy image so that subsequent image analysis is smoothly performed in step S905.

In addition, the controller 760 indicates the examination area in the picture of the stomach in step S909, and stores the duodenal bulb indication start time in step S910. Afterward, the controller 760 determines whether a lesion is detected in step S911, and indicates lesion information (see FIGS. 18A, 18B, and 18C) in the picture of the stomach when the lesion is detected in step S912.

Next, the controller 760 displays the examination time and the withdrawal time in step S913. Herein, the examination time refers to the time from the examination start time to the current (examination end) time, and the withdrawal time refers to the time from the duodenal bulb indication start time to the current (examination end) time.

In addition, the controller 760 determines whether to change the indication condition in step S914, and changes the indication condition when the changing of the indication condition is required in step S915. Herein, the controller 760 may transmit, to the image analysis part 740, an indication condition change command to indicate the gastroesophageal junction in the case of the upper gastric hernia or indicate the gastric cardia and the gastric fundus in the case of the paraesophageal hernia.

In addition, the controller 760 may transmit, to the image analysis part 740, an indication condition change command to differently indicate the hernia in the picture of the normal stomach, to not indicate the hernia in the picture of the normal stomach, or to report the gastric hernia (for example, the alarm or the indication of the region).

In the meantime, when no further gastroscopy image analysis is required as determined in step S903, the controller 760 stores the examination end time in step S920, and provides a result of analysis up to the current (examination end) time in step S921.

Hereinafter, additional description will be provided regarding the gastroscopy area indication system and method according to the present disclosure as described above.

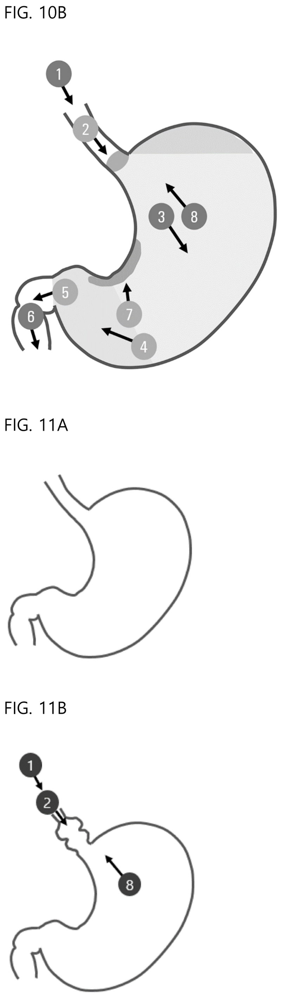

FIGS. 10A and 10B are diagrams illustrating a main area of the stomach.

Referring to FIGS. 10A and 10B, FIG. 10A shows the main area of the stomach, and FIG. 10B shows a general examination order (the photographing direction of a gastroscopy picture). A gastroscope is inserted through the esophagus and passes through the gastroesophageal junction and the gastric cardia, which are portions where the esophagus and the stomach are joined, and enters the inside of the stomach. The gastroscope inserted into the stomach captures the inside of the stomach in the following order : the gastric body → the gastric antrum → the duodenal bulb → the gastric angle → the gastric body → the gastric fundus → the gastric cardia. Herein, it is recommended to capture at least the gastroesophageal junction, the gastric antrum, the duodenal bulb, and the gastric angle and to store the captured images.

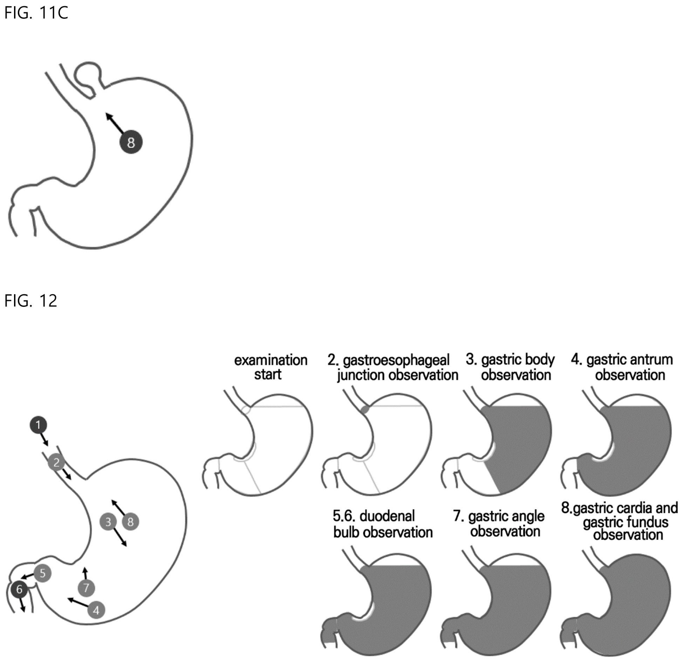

FIGS. 11A, 11B, and 11C are diagrams illustrating a lateral side of the stomach.

Referring to FIGS. 11A, 11B, and 11C, FIG. 11A shows the normal stomach, FIG. 11B shows the upper gastric hernia, and FIG. 11C shows the paraesophageal hernia. The upper gastric hernia shown in FIG. 11B is observed while moving from the esophagus to the stomach, and is also observed when making a U-turn depending on a degree of the hernia and examining the gastric cardia. The paraesophageal hernia shown in FIG. 11C is observed when making a U-turn of the gastroscope probe and examining the gastric cardia and the gastric fundus.

FIG. 12 is a diagram illustrating the display of an examination area of the stomach (in the case of the normal stomach).

FIG. 12 shows the case of indicating an examination area from a gastroscope probe insertion process. In general, the inside of the stomach is observed in the following order : examination start → gastroesophageal junction observation → gastric body observation → gastric antrum observation → duodenal bulb observation → gastric angle observation → gastric cardia and gastric fundus observation.

FIG. 13 is a diagram illustrating the display of an examination area of the stomach (in the case of the upper gastric hernia).

FIG. 13 shows the case of indicating an examination area from a gastroscope probe insertion process. Similarly to the case of the normal stomach shown in FIG. 12, the inside of the stomach is observed in the following order : examination start → gastroesophageal junction observation → gastric body observation → gastric antrum observation → duodenal bulb observation → gastric angle observation → gastric cardia and gastric fundus observation.

FIG. 14 is a diagram illustrating the display of an examination area of the stomach (in the case of the paraesophageal hernia).

FIG. 14 shows the case of indicating an examination area from a gastroscope probe insertion process. Similarly, the inside of the stomach is observed in the following order : examination start → gastroesophageal junction observation → gastric body observation → gastric antrum observation → duodenal bulb observation → gastric angle observation → gastric cardia and gastric fundus observation.

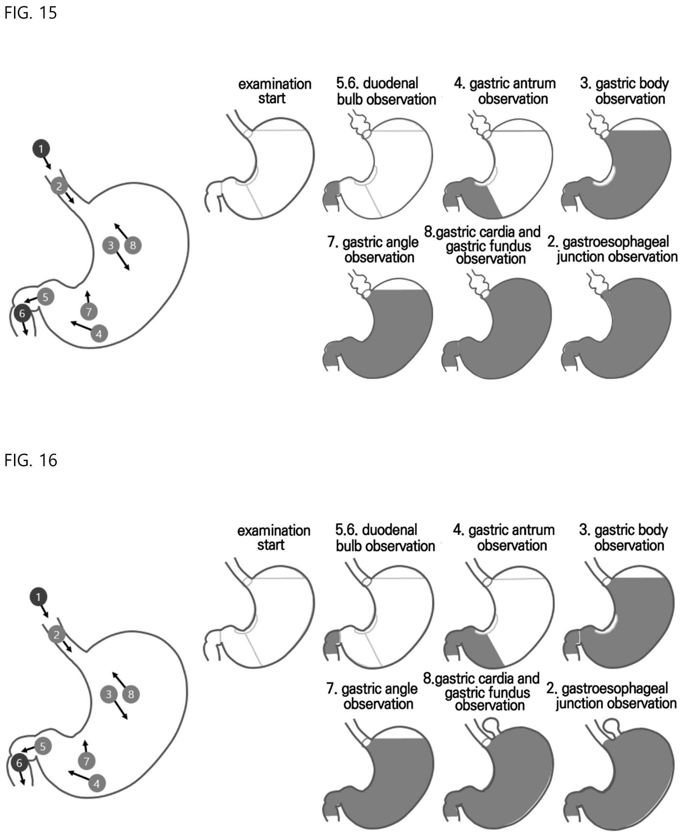

FIG. 15 is a diagram illustrating another example of the display of an examination area of the stomach (in the case of the upper gastric hernia).

FIG. 15 shows the case of indicating an examination area from a gastroscope probe withdrawal process. The inside of the stomach is observed in the following order : examination start → duodenal bulb observation → gastric antrum observation → gastric body observation → gastric angle observation → gastric cardia and gastric fundus observation → gastroesophageal junction observation (that is, in reverse order while the probe is withdrawn).

FIG. 16 is a diagram illustrating another example of the display of an examination area of the stomach (in the case of the paraesophageal hernia).

FIG. 16 shows the case of indicating an examination area from a gastroscope probe withdrawal process. Similarly to the case shown in FIG. 15, the inside of the stomach is observed in the following order : examination start → duodenal bulb observation → gastric antrum observation → gastric body observation → gastric angle observation → gastric cardia and gastric fundus observation → gastroesophageal junction observation (that is, in reverse order while the probe is withdrawn).

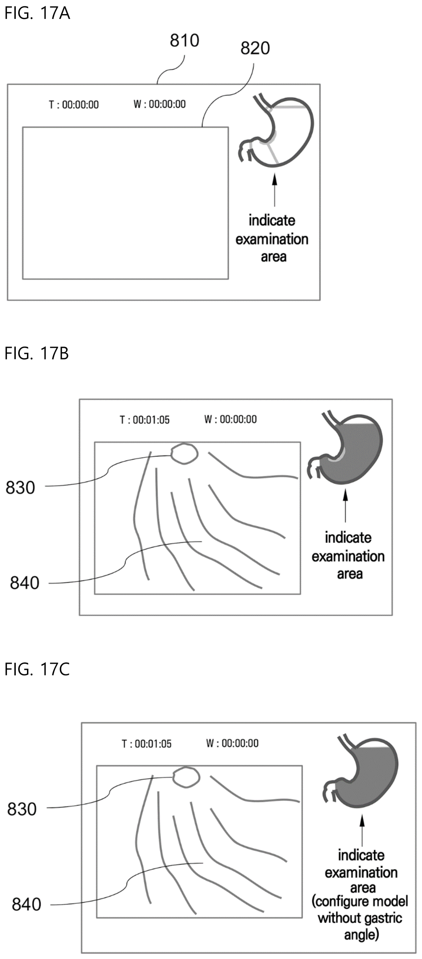

FIGS. 17A, 17B, and 17C are diagrams illustrating examples of providing a result of analysis (examples of an examination of the normal stomach).

Referring to FIGS. 17A, 17B, and 17C, these illustrate providing a result of analysis of a normal stomach examination in a gastroscope probe insertion process. FIG. 17A shows a gastroscopy start screen, and FIGS. 17B and 17C show screens illustrating examples of a gastric antrum examination. In particular. FIG. 17C shows a screen illustrating a state of indicating an examination area by a model configuration without the gastric angle. In FIGS. 17A to 17C, reference numeral 810 denotes a gastroscopy image analysis software screen, 820 denotes a gastroscopy image analysis region, 830 denotes the pylorus, and 840 denotes the gastric antrum. In addition, T denotes the examination time, and W denotes the withdrawal time.

FIGS. 18A, 18B, and 18C are diagrams illustrating examples of providing a result of analysis (examples of detection of an upper gastric hernia and a lesion).

Referring to FIGS. 18A, 18B, and 18C, these illustrate providing a result of analysis of detection of an upper gastric hernia and a lesion in a gastroscope probe insertion process. Similarly, FIG. 18A shows a gastroscopy start screen, and FIGS. 18B and 18C show screens illustrating examples of a gastric antrum examination. In particular. FIG. 18C shows a screen illustrating a state of indicating an examination area by a model configuration without the gastric angle. In FIGS. 18A, 18B, and 18C, reference numeral 810 denotes a gastroscopy image analysis software screen, 820 denotes a gastroscopy image analysis region, 830 denotes the pylorus, 840 denotes the gastric antrum, 850 denotes the upper gastric hernia, and 860 denotes the lesion. In addition, T denotes the examination time, and W denotes the withdrawal time.

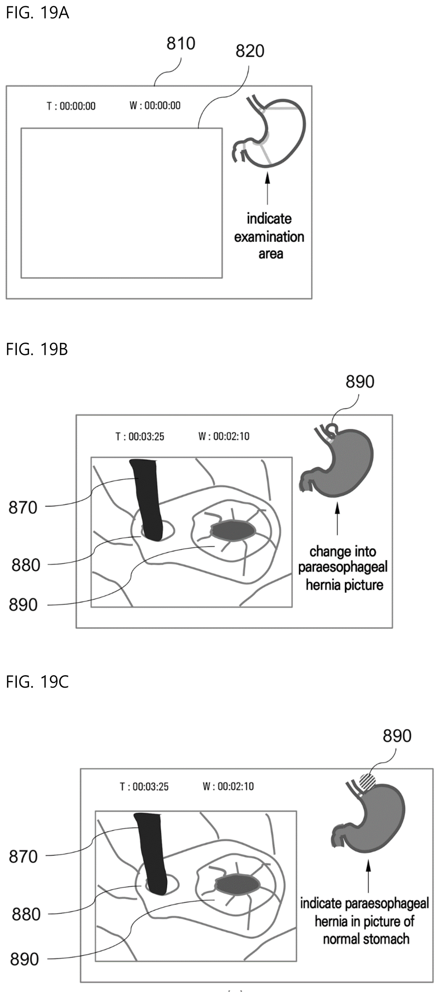

FIGS. 19A, 19B, and 19C are diagrams illustrating examples of providing a result of analysis (examples of an examination of the paraesophageal hernia).

Referring to FIGS. 19A, 19B, and 19C, these illustrate providing a result of analysis of a paraesophageal hernia examination in a gastroscope probe withdrawal process. Similarly, FIG. 19A shows a gastroscopy start screen, and FIGS. 19B and 19C show detection of a hernia when examining the gastric cardia and the gastric fundus while making a U-turn of the probe. FIG. 19B shows a state of change into a paraesophageal hernia picture, and FIG. 19C shows a state of indicating a paraesophageal hernia in the picture of the normal stomach. In FIGS. 19A, 19B, and 19C, reference numeral 810 denotes a gastroscopy image analysis software screen, 820 denotes a gastroscopy image analysis region, 870 denotes a probe, 880 denotes the gastric cardia, and 890 denotes the paraesophageal hernia. In addition, T denotes the examination time, and W denotes the withdrawal time.

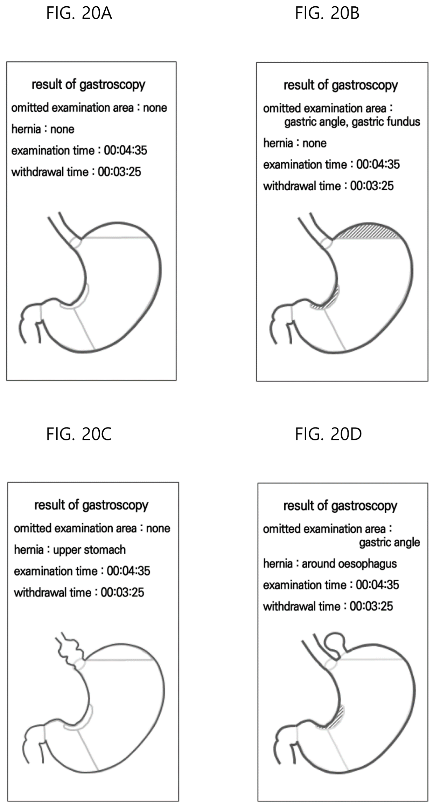

FIGS. 20A, 20B, 20C, and 20D are diagrams illustrating examples of providing a result of analysis (examples of result reports).

Referring to FIGS. 20A, 20B, 20C, and 20D, results of gastroscopy are shown. FIG. 20A shows a case in which there are no omitted examination area and no hernia. FIG. 20B shows a case in which omitted examination areas are the gastric angle and the gastric fundus and there is no hernia. FIG. 20C shows a case in which there is no omitted examination area and there is an upper gastric hernia. FIG. 20D shows a case in which an omitted examination area is the gastric angle and there is a paraesophageal hernia.

As described above, a gastroscopy area indication system and method according to the present disclosure indicate a normal stomach and a herniated stomach in a screen by distinguishing therebetween, thereby specifically indicating a stomach examination area, and inform an examiner of a hernia, thereby enabling a more thorough gastroscopy.

In addition, by indicating a main stomach examination area, it is possible to check areas examined up to the present, to check examination-omitted positions after completion of the examination, and to provide hernia information.

Although an exemplary embodiment of the present disclosure has been described in detail, the present disclosure is not limited thereto, and it is obvious to those skilled in the art that various modification and applications can be made within the scope of the technical idea of the present disclosure. Accordingly, the true scope of the present disclosure should be interpreted by the following claims, and all technical ideas within the scope equivalent thereto should be interpreted as being included in the scope of the present disclosure.

## Claims

1. A gastroscopy area indication system, comprising:
a model loading/condition setting part configured to load a gastroscopy image analysis model, and set an analysis condition of the analysis model;
an image receiving part configured to receive a gastroscopy image frame;
an image preprocessing part configured to preprocess a gastroscopy image received through the image receiving part so that subsequent image analysis is smoothly performed;
an image analysis part configured to analyze the gastroscopy image preprocessed by the image preprocessing part by using the image analysis model based on artificial intelligence (AI), and detect and indicate, on the basis of a result of analysis, at least one selected from a group of an examination area, a hernia area, and a lesion area in the gastroscopy image; and
a controller configured to check states and control operations of the model loading/condition setting part, the image receiving part, the image preprocessing part, and the image analysis part, and initialize an analysis screen and displays a picture of a normal stomach when the model loading/condition setting part completes loading of the gastroscopy image analysis model and setting of the analysis condition of the analysis model, and provide the result of analysis performed by the image analysis part.

2. The gastroscopy area indication system of claim 1, wherein preprocessing of the gastroscopy image by the image preprocessing part includes analysis region cropping and input size adjustment.

3. The gastroscopy area indication system of claim 1, wherein the image analysis model of the image analysis part is configured to include an examination area detection model for detecting the examination area, a hernia detection model for detecting the hernia area, and a lesion detection model for detecting the lesion area.

4. The gastroscopy area indication system of claim 3, wherein the lesion detection model has a lesion attribute identification function for determining whether a lesion is benign or malignant.

5. The gastroscopy area indication system of claim 1, wherein in detecting and indicating at least one selected from the group of the examination area, the hernia area, and the lesion area in the gastroscopy image by the image analysis part, the examination area includes a gastroesophageal junction, a gastric body, a gastric antrum, a gastric cardia, a gastric angle, a gastric fundus, and a duodenal bulb, and the hernia area includes an upper gastric hernia and a paraesophageal hernia.

6. The gastroscopy area indication system of claim 1, wherein in detecting and indicating at least one selected from the group of the examination area, the hernia area, and the lesion area in the gastroscopy image by the image analysis part, the controller is configured to transmit, to the image analysis part, an indication condition change command to indicate a gastroesophageal junction in a case of an upper gastric hernia, or indicate a gastric cardia and gastric fundus in a case of a paraesophageal hernia.

7. The gastroscopy area indication system of claim 1, wherein in detecting and indicating at least one selected from the group of the examination area, the hernia area, and the lesion area in the gastroscopy image by the image analysis part, the controller is configured to transmit, to the image analysis part, an indication condition change command to differently indicate a hernia in the picture of the normal stomach, or not indicate the hernia in the picture of the normal stomach, or report a gastric hernia.

8. A gastroscopy area indication system, comprising:
a model loading/condition setting part configured to load a gastroscopy image analysis model and a speech keyword recognition model, and set an analysis condition of the analysis model;
an image receiving part configured to receive a gastroscopy image frame;
an image preprocessing part configured to preprocess a gastroscopy image received through the image receiving part so that subsequent image analysis is smoothly performed;
an image analysis part configured to analyze the gastroscopy image preprocessed by the image preprocessing part by using the image analysis model based on artificial intelligence (AI), and detect and indicate, on the basis of a result of analysis, at least one selected from a group of an examination area, a hernia area, and a lesion area in the gastroscopy image;
a speech recognition part configured to read audio from a buffer storing the audio while the image analysis part performs the image analysis, and analyze the audio using the speech keyword recognition model based on AI, and recognize a speech keyword on the basis of the result of analysis and transmit the speech keyword to the image analysis part; and
a controller configured to check states and control operations of the model loading/condition setting part, the image receiving part, the image preprocessing part, the image analysis part, and the speech recognition part, and initialize an analysis screen and display a picture of a normal stomach when the model loading/condition setting part completes loading of the gastroscopy image analysis model and setting of the analysis condition of the analysis model, and provide the result of analysis performed by the image analysis part, wherein the result of analysis is provided by linking an analysis target detected by the image analysis model with a speech command (keyword) related to the analysis target spoken by an examiner.

9. The gastroscopy area indication system of claim 8, wherein preprocessing of the gastroscopy image by the image preprocessing part includes analysis region cropping and input size adjustment.

10. The gastroscopy area indication system of claim 8, wherein the image analysis model of the image analysis part is configured as a single image analysis model for detecting the examination area and the hernia area.

11. The gastroscopy area indication system of claim 8, wherein the image analysis model of the image analysis part is configured to include an examination area detection model for detecting the examination area, a hernia detection model for detecting the hernia area, and a lesion detection model for detecting the lesion area.

12. The gastroscopy area indication system of claim 11, wherein the lesion detection model has a lesion attribute identification function for determining whether a lesion is benign or malignant.

13. The gastroscopy area indication system of claim 8, wherein in detecting and indicating at least one selected from the group of the examination area, the hernia area, and the lesion area in the gastroscopy image by the image analysis part, the examination area includes a gastroesophageal junction, a gastric body, a gastric antrum, a gastric cardia, a gastric angle, a gastric fundus, and a duodenal bulb, and the hernia area includes an upper gastric hernia and a paraesophageal hernia.

14. The gastroscopy area indication system of claim 8, wherein in detecting and indicating at least one selected from the group of the examination area, the hernia area, and the lesion area in the gastroscopy image by the image analysis part, the controller is configured to transmit, to the image analysis part, an indication condition change command to indicate a gastroesophageal junction in a case of an upper gastric hernia, or indicate a gastric cardia and gastric fundus in a case of a paraesophageal hernia.

15. The gastroscopy area indication system of claim 8, wherein in detecting and indicating at least one selected from the group of the examination area, the hernia area, and the lesion area in the gastroscopy image by the image analysis part, the controller is configured to transmit, to the image analysis part, an indication condition change command to differently indicate a hernia in the picture of the normal stomach, or not indicate the hernia in the picture of the normal stomach, or report a gastric hernia.
